# EUROPEAN PATENT APPLICATION

(11) **EP 1 356 798 A1**
(43) Date of publication of application: **29.10.2003**
(21) Application number: 02252954.9
(22) Date of filing: 25.04.2002
(51) Int. Cl.: A61F 13/42

(54) **Wetness indicator for disposable diaper**

(71) Applicant: Chen, Chin-Chen, Pa Te City, Taoyuan Hsien (TW); Chang, Chien-Hsing, Pa Te City, Taoyuan Hsien (TW); Lu, Chia-Jung, Taoyuan (TW); Huang, Chiao-Heng, Sydney, N.S.W. 2074 (AU)
(72) Inventor: Chen, Chin-Chen, Pa Te City, Taoyuan Hsien (TW); Chen, Hong-lin, Pa Te City, Taoyuan Hsien (TW); Chen, Hong-Ming, Pa Te City, Taoyuan Hsien (TW); Chen, Yi-Ran, Pa Te City, Taoyuan Hsien (TW); Chen, Hong-Chih, Pa Te City, Taoyuan Hsien (TW)
(74) Representative: Needle, Jacqueline

(57) **Abstract**

An automatic diaper wetness warning device includes a diaper (10), a wetness signal transmitting device (20) and an externally disposed signal receiver. The wetness signal transmitting device (20) includes a signal transmitter (21) and a positive and negative pole sensing electric wire assembly (22). A suitable length of the inner end of the wire assembly (22) is provided with an insulation coating material (222) which can conduct electricity when wet, and is wound into a signal actuating section (223) embedded in the diaper in a position that can readily absorb urine. When the insulation coating material (222) of the signal actuating section (223) is wet it is short circuited and the signal transmitter (21) generates a signal which is received by the signal receiver. The signal receiver generates a suitable warning to remind a person taking care of the user of the diaper to change diaper.

## Description

The present invention relates to an automatic diaper wetness warning device, for example to an automatic warning device which, when a diaper is wet, sends a warning to change the diaper.

Disposable diapers provide great convenience in looking after babies and adults with incontinence. Wet diapers are uncomfortable to the users and the urine may leak, which may mean more work to persons looking after the users.

Conventional diapers are not provided with any wetness warning device. Therefore, the diapers have to be checked from time to time, which may disturb the users, especially in cold weather.

Although there are diapers with wetness indicating means that change color when the diapers are wet, it is still necessary to pull down the user's pants to check the diapers. If the diapers are not checked in time, leakage may also occur.

It is an object of the present invention to provide improvements to the prior art.

According to the present invention there is provided an automatic diaper wetness warning device comprising: a diaper; a wetness signal transmitting device including: a signal transmitter having a control circuit board, said control circuit board including a power source and an electrical connector connected thereto; a first electrical conductor connected to a positive pole of the electrical connector; and a second electrical conductor connected to a negative pole of the electrical connector, said first and second electrical conductors each having a first end and a second end, said first ends of said first and second electrical conductors being provided with a connecting device for connecting with said electrical connector, said second end of each of said first and second electrical conductors being disposed in said diaper and having an insulation material on portions of second ends which conducts electricity between said first and second electrical conductors when said insulation material is wet such that said signal transmitter transmits a signal; and a signal receiver for receiving signals transmitted by said transmitter and subsequently transmitting a warning signal; wherein said power source of said signal transmitter cuts off within a period of time after completion of transmission of warning signals from said signal transmitter.

An embodiment of the invention has the advantage that a wetness signal transmitting device is provided such that when the diaper is wet, the wetness signal transmitting device sends a signal which is received by a signal receiver disposed externally. The signal receiver also sends a warning signal to remind persons looking after users of the diapers to change the diaper.

Embodiments of the invention also enable the wetness signal transmitting device and the signal receiver to be adjusted to send or receive signals of different frequencies so that the warning device can be used for a number of users at the same time and can help persons looking after the users to identify which particular user's diapers are wet.

In an embodiment, the wetness signal transmitting device can send two different kinds of signals to remind persons looking after users of the diapers to know the diaper is in a little wet condition or in a high wet condition.

Embodiments of the present invention will hereinafter be described, by way of example, with reference to the accompanying drawings, in which,

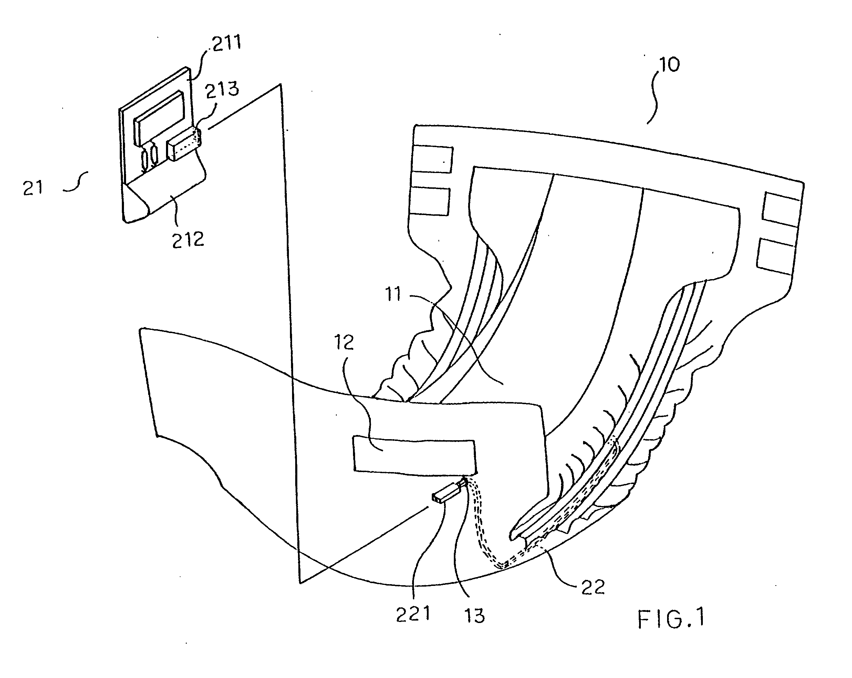

Fig. 1 is an exploded perspective view of an automatic wetness warning device of the invention;

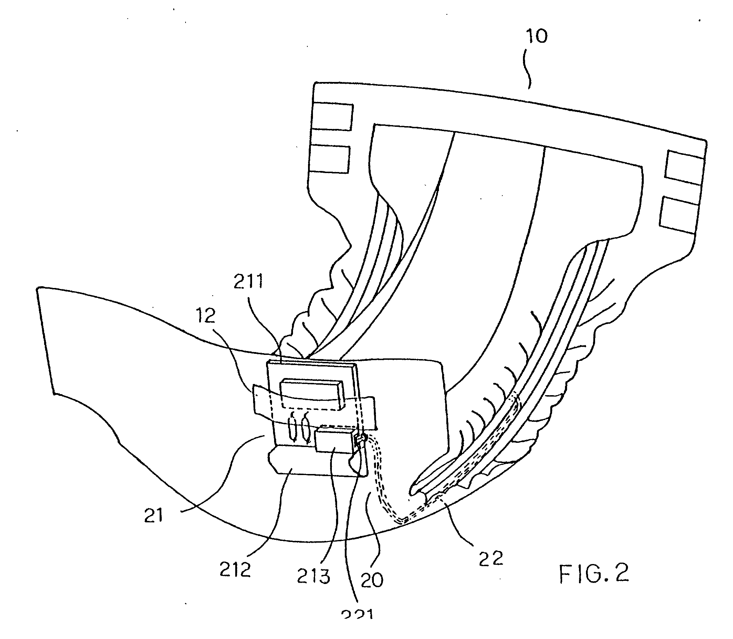

Fig. 2 shows an assembled perspective view of an automatic wetness warning device of the present invention;
Fig. 3 is a schematic view of an embodiment, illustrating embedding of a signal actuating section;
Fig. 3A is a schematic enlarged view of the signal actuating section of Fig. 3;
Fig. 4 is a schematic view of a second embodiment of a signal actuating section;
Fig. 5 is an exploded perspective view of a further embodiment of an automatic wetness warning device of the present invention;
Fig. 6 is a schematic view of the embodiment of the signal actuating section shown in Fig. 5; and
Fig 7 is a schematic view of a still further embodiment of a signal actuating section.

With reference to the drawings, an automatic diaper wetness warning device of the invention includes a diaper 10, a wetness signal transmitting device 20 and an externally disposed signal receiver (not shown).

The diaper 10 is a disposable diaper worn by a user to absorb the urine or feces.

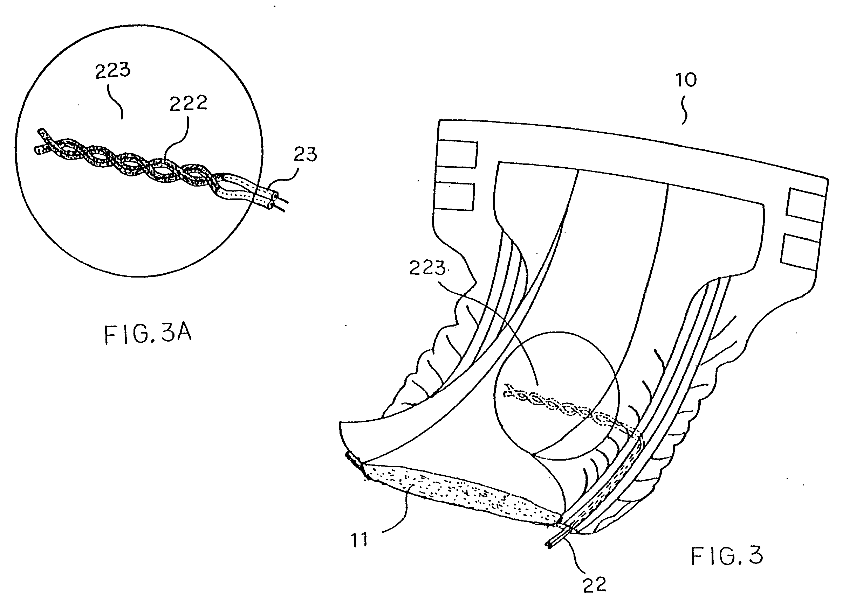

The wetness signal transmitting device 20 includes a signal transmitter 21 and a positive and negative pole sensing electric wire assembly 22. The signal transmitter 21 has a specially devices control circuit board 211. The control circuit board 211 is provided with a power source 212 formed by a battery and a socket 213 at suitable positions. The wire assembly 22 has an outer surface provided with an insulation material 23 and is disposed on the diaper 10 at a suitable position such that one end thereof extends through a hole 13 formed in the diaper 10 at a suitable position and is provided with a plug 221 corresponding to the socket 213 for connecting the wire assembly 22 and the signal transmitter 21. A suitable length of the other end of the wire assembly 22 is provided with an insulation coating material 222 that is electrically conductive when wet (such as a water absorbing paper), which is wound into a signal actuating section 223, as shown in Fig. 3 or Fig. 3 A, and is embedded in the diaper 10 in a position that can absorb urine.

The signal receiver can receive signals sent by the signal transmitter 21 and send a suitable warning signal (such as light, sound or a combination of both) to a person taking care of the user of the diaper. When the insulation coating material 222 provided on the signal actuating section 223 is wet by urine and becomes short-circuited, the signal transmitter 21 of the wetness signal transmitting device 20 will automatically send a suitable signal, which is received by the signal receiver disposed externally. The signal receiver then sends a warning signal to remind the person taking care of the user to change the diaper.

The diaper 10 is optionally provided with a self-adhesive tape 12 at a position corresponding to the plug 221 for securing the signal transmitter of the wetness signal transmitting device 20. Since the wetness signal transmitting device 20 utilizes the socket 213 thereof to couple with the plug 221 of the wire assembly 22, once the diaper 10 is wet and has to be disposed, the signal transmitter 21 can be removed and inserted into a new diaper with the wire assembly 22. In addition, since the signal transmitter 21 is very small, it will not cause any discomfort to the user, and is easy to operate.

To prevent consumption of electric energy of the power source 212 after short-circuit of the signal actuating section 223, the power source 212 of the wetness signal transmitting device 20 can be cut off in a suitable period of time after short-circuit of the signal transmitting section 223. When the signal transmitter 21 is changed to a new diaper, the circuit of the power source 212 will be automatically opened to supply the necessary power.

In addition, in order for the present invention to be adapted for use in nurseries or homes for the aged where users of diapers are many, the signal transmitter 21 and the signal receiver can be adjusted to be able to send or receive signals of varying frequencies so as to warn persons taking care of particular users when their diapers are wet, without disturbing other users. Thus, the work of checking the diapers can be made easier.

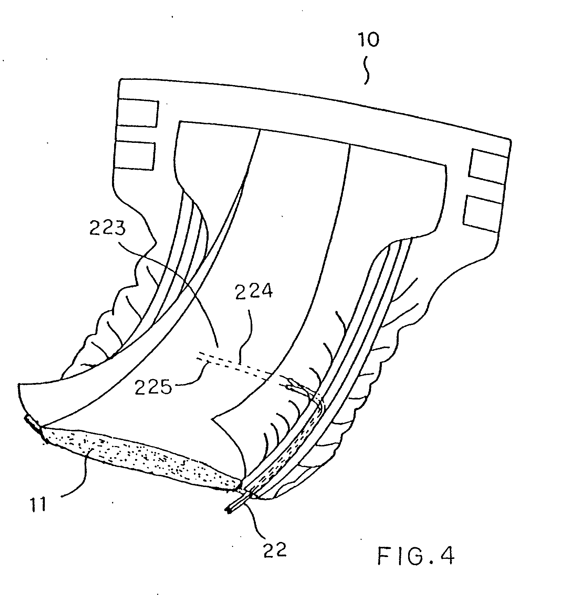

Optionally, the signal actuating section 223 dose not include the insulation coating material 222. In other words, wires 224 and 225 can be spacedly embedded in the water absorbing layer 11 of the diaper 10, as shown in Fig. 4. When the water absorbing layer 11 is wet, the wires 224, 225 will, due to conductive capability of the urine, activate the signal transmitter 21 at the other end thereof to warn wetness of the diaper.

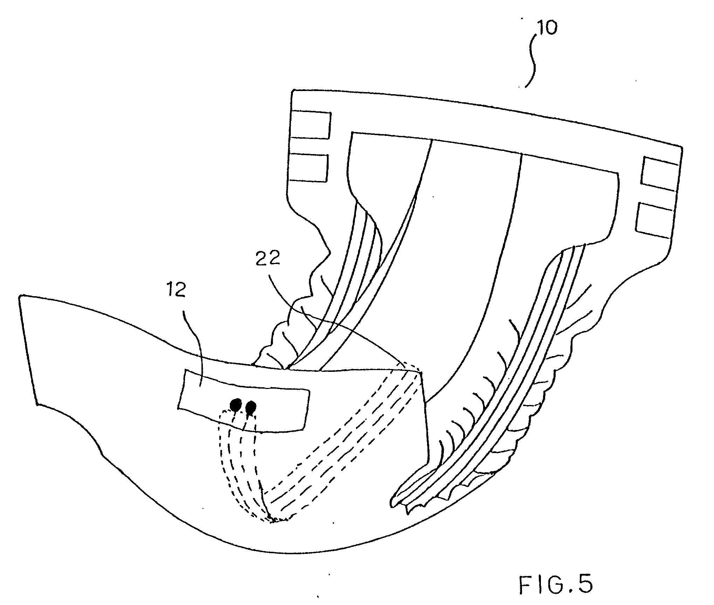

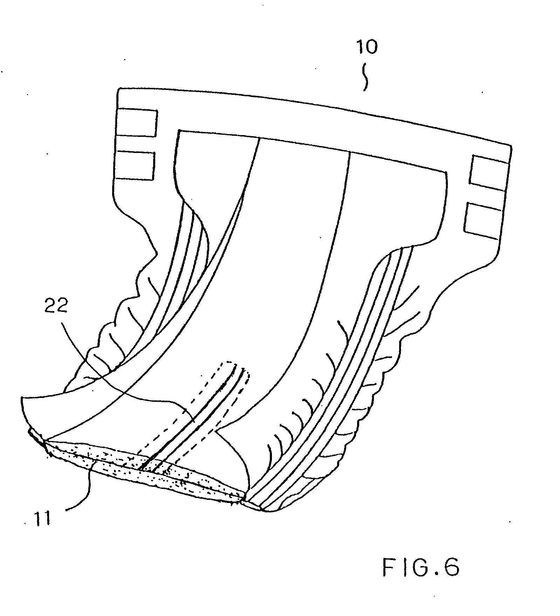

Of course, like a printed-circuit, the said wire assembly 22 can also be formed on one suitable soft strip as shown in Fig. 5 and Fig. 6, i.e. the third embodiment of the present invention. Additionally, for letting said printed-circuit type wire assembly 22 be connected with said signal transmitter 21 directly, we delete said socket 213 and plug 223, and let said printed-circuit type wire assembly be connected to a signal receiving end of said control circuit board 211 directly.

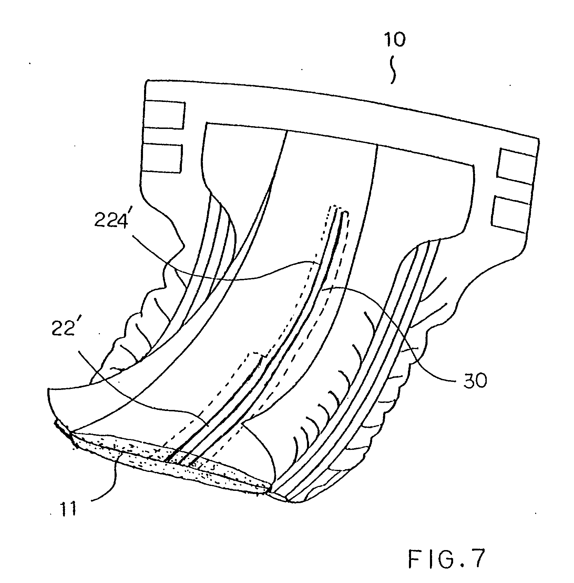

Fig. 7 illustrates the fourth embodiment of the present invention. Here, one of said wire assembly 22' is forwardly extended into a extension section 224'. Further, the side of said wire assembly 22' was provided with a third electrical conductor 30. One end of said third electrical conductor 30 is disposed closely with the end of said extension section 224', and another end of said third electrical conductor 30 is also connected with said signal transmitter21. As above described, electricity will be conducted between said wire assembly 22 when said diaper is in low wet condition such that said signal transmitter 21 will transmit a first signal (like green light). For avoiding waste of the diaper, the said low wet diaper maybe doesn't need to be changed at that condition. Because the position of the ends of said extension section 224' and said third electrical conductor 30 are higher, so, if the diaper is in full wet condition, the electricity will be conducted between said extension section 224' and said third electrical conductor 30 such that said signal transmitter 21 will transmit a second signal (like red light) to remind the person taking care of the user to change the diaper.

Further, for promoting the efficiency of conduction, there will be a conducting layer disposed in a portion of said diaper. The said conducting layer has a set of first conducting wires and a set of second conducting wires formed on a suitable soft layer. Because said printed-circuit type wire assembly was connected to said set of first conducting wires and said set of second conducting wires separately, so, it can promote the efficiency of conduction.

In summary, since the automatic diaper wetness warning device of the present invention gives an indication of diaper wetness to the person taking care of the user of the diaper, changing of the diaper can be done without taking the trouble to check the diaper, which may disturb the user.

Although the present invention has been illustrated and described with reference to an embodiment thereof, it should be understood that it is in no way limited to the details of such embodiment but is capable of numerous modifications within the scope of the invention.

## Claims

1. An automatic diaper wetness warning device comprising:
a diaper;
a wetness signal transmitting device including:
a signal transmitter having a control circuit board, said control circuit board including a power source and an electrical connector connected thereto;
a first electrical conductor connected to a positive pole of the electrical connector; and
a second electrical conductor connected to a negative pole of the electrical connector, said first and second electrical conductors each having a first end and a second end, said first ends of said first and second electrical conductors being provided with a connecting device for connecting with said electrical connector, said second end of each of said first and second electrical conductors being disposed in said diaper and having an insulation material on portions of second ends which conducts electricity between said first and second electrical conductors when said insulation material is wet such that said signal transmitter transmits a signal; and
a signal receiver for receiving signals transmitted by said transmitter and subsequently transmitting a warning signal;
wherein said power source of said signal transmitter cuts off within a period of time after completion of transmission of warning signals from said signal transmitter.

2. An automatic diaper wetness warning device as claimed in Claim 1,
wherein said insulation material is a water absorbing paper or a suitable water absorbing material.

3. An automatic diaper wetness warning device comprising:
a diaper;
a wetness signal transmitting device including:
a signal transmitter having a control circuit board, said control circuit board including a power source and an electrical connector connected thereto;
a first electrical conductor connected to a positive pole of the electrical connector; and
a second electrical conductor connected to a negative pole of the electrical connector, said first and second electrical conductors each having a first end and a second end, said first ends of said first and second electrical conductors being provided with a connecting device for connecting with said electrical connector, said second end of each of said first and second electrical conductors being disposed in a portion of said diaper such that electricity is conducted between said first and second electrical conductors when said portion of said diaper is wet such that said signal transmitter transmits a signal; and
a signal receiver for receiving signals transmitted by said transmitter and subsequently transmitting a warning signal;
wherein said power source of said signal transmitter cuts off within a period of time after completion of transmission of warning signals from said signal transmitter.

4. An automatic diaper wetness warning device comprising:
a diaper;
a wetness signal transmitting device including:
a signal transmitter having a control circuit board, said control circuit board including a power source and a signal receiving end thereto;
a first electrical conductor and a second electrical conduct formed on a suitable soft strip with a suitable space and each having a first end and a second end, said first ends of said first and second electrical conductors being separately connected with a positive pole and a negative pole of the signal receiving end, said second end of each of said first and second electrical conductors being disposed in a portion of said diaper such that electricity is conducted between said first and second electrical conductors when said portion of said diaper is wet such that said signal transmitter transmits a signal; and
a signal receiver for receiving signals transmitted by said transmitter and subsequently transmitting a warning signal;
wherein said power source of said signal transmitter cuts off within a period of time after completion of transmission of warning signals from said signal transmitter.

5. An automatic diaper wetness warning device as claimed in Claim 4, further comprising a conducting layer formed on a suitable soft layer and having a set of first conducting wires for connecting with the second end of said first electrical conductor and a set of second conducting wires for connecting with the second end of said second electrical conductor and being disposed in a portion of said diaper.

6. An automatic diaper wetness warning device as claimed in Claim 4,
wherein the second end of said second electrical conductor further forwardly extends to a suitable position, further, the side of said second electrical conductor was provided with a third electrical conductor having a first end and a second end, the first end of said third electrical conductor is also connected with said signal transmitter, and the second end of said third electrical conductor was disposed on the side of the second end of said second electrical conductor such that electricity is conducted between said second and third electrical conductors when said diaper is in full wet condition such that said signal transmitter transmits a signal different from that when electricity is conducted between said first and second electrical conductors when said diaper is in low wet condition.

7. An automatic diaper wetness warning device as claimed in any preceding claim, further comprising a self-adhesive tape at the connecting device for securing said signal transmitter to said diaper.

8. An automatic diaper wetness warning device as claimed in any preceding claim, wherein the transmitting frequency of said wetness signal transmitting device is adjustable, and said signal receiver is capable of receiving one or more than one kind of frequency transmitted from one or more than one wetness signal transmitting device at the same time.

9. An automatic diaper wetness warning device as claimed in any preceding claim, wherein said warning signal sent by said signal receiver is one of a warning light, a warning sound, and a combination of a warning light and a warning sound.

10. A diaper for use in the automatic diaper wetness warning device comprising an outer water-impermeable layer, an inner sheet of a water-permeable film and several water absorbing layers, **characterized in that**: said water absorbing layers was provided with a first electrical conductor and a second electrical conductor with a suitable space, said first electrical conductor and second electrical conductor have a first end and a second end separately, the second ends of said first electrical conductor and said second electrical conductor are disposed in the suitable position of said water absorbing layers, and, the first ends of said first electrical conductor and said second electrical conductor are extended to a suitable position of outer side of said outer water-impermeable layer.

11. A diaper as claimed in Claim 10, wherein the second end of said second electrical conductor further forwardly extends to a suitable position, further, the side of said second electrical conductor was provided with a third electrical conductor having a first end and a second end, the second end of said third electrical conductor was disposed on the side of the second end of said second electrical conductor, and, the first end of said third electrical conductor is also extended to a suitable position of outer side of said outer water-impermeable layer.
